(19) Europäisches Patentamt
European Patent Office
Office européen des brevets

(11) Veröffentlichungsnummer: **0 059 168**
**B1**

(12) # EUROPÄISCHE PATENTSCHRIFT

(45) Veröffentlichungstag der Patentschrift: **02.10.85**

(51) Int. Cl.⁴: **C 07 C 149/20**, C 08 K 5/37, C 10 M 135/26

(21) Anmeldenummer: **82810070.1**

(22) Anmeldetag: **15.02.82**

(54) **Organische Elastomere und mineralische und synthetische Schmieröle, enthaltend Phenol-mercaptocarbonsäureester als Stabilisatoren.**

(30) Priorität: **19.02.81 CH 1107/81**

(43) Veröffentlichungstag der Anmeldung: **01.09.82 Patentblatt 82/35**

(45) Bekanntmachung des Hinweises auf die Patenterteilung: **02.10.85 Patentblatt 85/40**

(84) Benannte Vertragsstaaten: **BE DE FR GB IT NL**

(56) Entgegenhaltungen:
**FR - A - 1 560 259**
**US - A - 3 546 272**
**US - A - 3 789 064**
**US - A - 3 810 869**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Postfach, CH-4002 Basel (CH)**

(72) Erfinder: **Rosenberger, Siegfried, Im Gehracker 11, CH-4125 Riehen (CH)**
Erfinder: **Schwarzenbach, Kurt, Dr., Im Noll 34, CH-4148 Pfeffingen (CH)**

**EP 0 059 168 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft die Verwendung von bestimmten Phenol-mercaptocarbonsäureestern zum Stabilisieren von Elastomeren und Schmiermitteln.

Als Stand der Technik ist die DE-PS 1 288 604 anzuführen, durch die Phenolmercaptocarbonsäureester und ihre Verwendung als Stabilisatoren in sehr breiter Form bereits beansprucht werden. Diese Phenol-mercaptocarbonsäureester entsprechen der Formel a oder b:

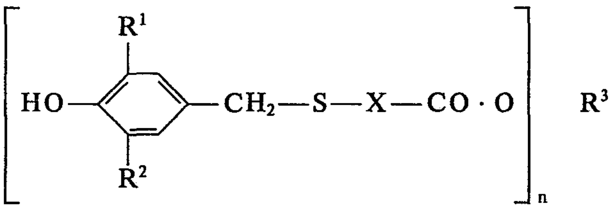

(a)

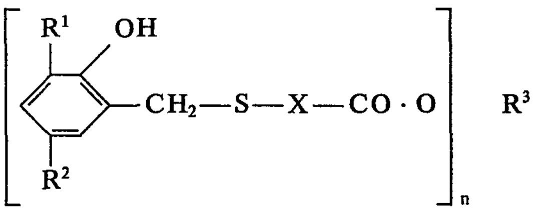

(b)

worin n eine ganze Zahl von 1 bis 4, $R^1$ und $R^2$ gleiche oder verschiedene, geradkettige oder verzweigte Alkylgruppen mit 1 bis 4 C-Atomen, $R^3$ eine gerade, verzweigte oder cyclische Alkylgruppe, Benzyl-, Thioäther- oder Äthergruppe (wenn $n = 1$ ist) bzw. Alkylengruppe (wenn $n = 2$ bis 4 ist) mit insgesamt 1 bis 20 C-Atomen und X eine geradkettige oder verzweigte Alkylengruppe mit 1 oder 2 C-Atomen bedeuten.

In dieser Patentschrift werden ganz deutlich die Verbindungen der Formel (a), worin $R^1$ und $R^2$ Methyl bedeuten, als besonders vorteilhaft in ihrer Wirkung als Stabilisatoren hervorgehoben.

Das US-Patent 3 810 869 beschreibt ebenfalls Phenolmercaptocarbonsäureester in sehr breiter Form als Stabilisatoren für organisches Material wie z. B. Benzine, Kautschuk, Polyolefine. Das US-Patent 3 789 064 offenbart ganz generisch S-haltige Ester von substituierten Hydroxyphenylcarbonsäuren und ihre Verwendung als Antioxidantien für organisches Material ganz allgemein. Aus keinem der beiden US-Patenten geht jedoch hervor, daß sich spezielle Phenolmercaptocarbonsäureester durch eine unerwartet hohe Wirksamkeit als Antioxidantien in ganz bestimmten Substraten auszeichnen.

Es wurde aber gefunden, daß ganz bestimmte Ester der durch die obigen Patentschriften generisch offenbarten Produktegruppe und zwar, entgegen der aus der DE-PS 1 288 604 gewonnenen Lehre, nicht die in beiden ortho-Stellungen zur OH-Gruppe mit Methyl substituierten Phenolmercaptocarbonsäureester, in Elastomeren und Schmiermitteln in ihrer Wirkung als Stabilisatoren überraschend vorteilhaft herausragen.

Gegenstand der Erfindung sind demnach organische Elastomere sowie mineralische und synthetische Schmieröle, enthaltend als Stabilisator einen Phenol-mercaptocarbonsäureester der Formel I

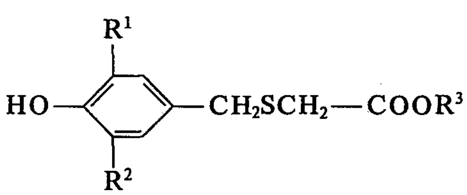

(I)

worin $R^1$ tert.-Butyl, $R^2$ Methyl oder tert.-Butyl und $R^3$ geradkettiges oder verzweigtes, ununterbrochenes oder durch $-O-$ oder $-S-$ unterbrochenes $C_4-C_{18}$-Alkyl, $C_5-C_6$-Cycloalkyl oder Benzyl bedeuten.

$R^3$ kann beispielsweise die folgenden Reste bedeuten: Isobutyl, n-Hexyl, n-Octyl, 2-Äthylhexyl, n-Decyl, n-Dodecyl, n-Octadecyl, 3-Thia-heptyl, 3-Thia-5-methylhexyl.

Gemäß der Erfindung werden insbesondere die folgenden synthetischen Elastomeren stabilisiert:

a) Polydiene, vorzugsweise Polybutadien, Polyisopren oder Polychloropren.
b) Blockpolymerisate, vorzugsweise die Typen Styrol-Butadien-Styrol, Styrol-Isopren-Styrol oder Styrol-(Äthylen-Propylen)-Styrol.
c) Acrylnitril-Butadien-Polymere.

Die Verbindungen der Formel II

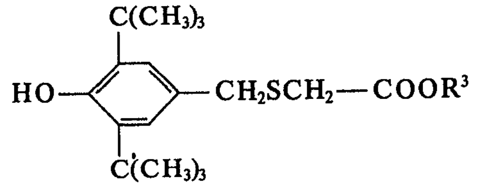

(II)

worin $R^3$ $C_5-C_{12}$-Alkyl bedeutet, sind besonders gut als Stabilisatoren für obengenannte Polymere und für Schmiermittel gegen Abbau durch Sauerstoff und Wärme geeignet.

Besonders gut für die Verwendung als Stabilisator gemäß der Erfindung ist die Verbindung der Formel III

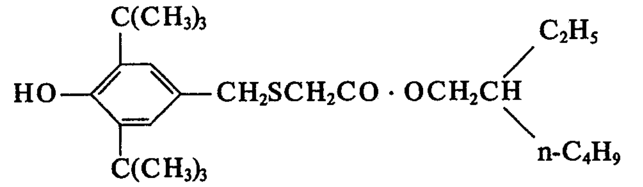

(III)

geeignet.

Im allgemeinen werden die erfindungsgemäß verwendeten Stabilisatoren dem zu stabilisierenden Material in Mengen von 0,01 bis 10%, vorzugsweise 0,05 bis 0,5%, bezogen auf das Gesamtgewicht des zu stabilisierenden Materials, zugesetzt.

Die zu stabilisierenden Elastomeren liegen gegebenenfalls als Latices vor und können als solche stabilisiert werden.

Als weitere Additive, mit denen zusammen die erfindungsgemäß verwendbaren Stabilisatoren eingesetzt werden können, sind beispielsweise zu nennen:

2,6-Di-tert.-butyl-4-methylphenol,
4,4'-Thio-bis-(6-tert.-butyl-3-methylphenol),
2,2'-Methylen-bis-(6-tert.-butyl-4-methylphenol),
2,2'-Methylen-bis-(4-methyl-6-cyclohexylphenol),
1,1-Bis-(5-tert.-butyl-4-hydroxy-2-methylphenyl)-butan,
1,3,5-tri-(3,5-di-tert.-butyl-4-hydroxy-benzyl)-2,4,6-trimethylbenzol,
Di-(3,5-di-tert.-butyl-4-hydroxybenzyl)-sulfid,
Stearyl-3(3,5-di-tert.-butyl-4-hydroxyphenyl)-propionat,
1,6-Hexandiol-bis-3-(3,5-di-tert.-butyl-4-hydroxyphenyl)-propionat,
Tris-(nonylphenyl)phosphit, Distearoyl-pentaerythrit-diphosphit,
Tris-(2,4-di-tert.-butylphenyl)-phosphit,
Di-(2,4-di-tert.-butyl-phenyl)-pentaerythrit-diphoshit,
Tetrakis-(2,4-di-tert.-butylphenyl)-4,4'-biphenylylen-diphosphonit.

Die Herstellung der Verbindungen der Formel I ist bekannt und beispielsweise bereits in der DE-PS 1 288 604 beschrieben worden.

Die erfindungsgemäß verwendeten Stabilisatoren können auch zusammen mit anderen bekannten Additiven eingesetzt werden. So sind beispielsweise die folgenden Substanzen anzuführen:

Alkylierte Monophenole, alkylierte Hydrochinone, hydroxylierte Thiodiphenyläther, Alkyliden-Bisphenole, Hydroxybenzylverbindungen, Acylaminophenole, Ester der 3-(3,5-di-tert.-butyl-4-hydroxyphenyl)-propionsäure, Amide der 3-(3,5-di-tert.-butyl-4-hydroxyphenyl)-propionsäure, 2-(2'-Hydroxyphenyl)-benztriazole, 2-Hydroxy-benzophenone, Nickelverbindungen, sterisch gehinderte Amine, Phosphite, z. B. Tris-(nonylphenyl)-phosphit, Distearoyl-pentaerythrit-diphosphit, Tris-(2,4-di-tert.-butylphenyl)-phosphit, Di-(2,4-di-tert.-butyl-phenyl)-pentaerythrit-diphosphit, Tetrakis-(2,4-di-tert.-butylphenyl)-4,4'-biphenylylen-diphosphonit.

Die Verbindungen der Formel I wirken schon in sehr geringen Mengen als Antioxidantien, Antikorrosionsmittel und Hochdruck-Zusätze in Schmiermitteln. So zeigen mineralische und synthetische Schmieröle, sowie deren Gemische, welche 0,001 bis 5 Gew.-% bezogen auf das Schmiermittel und vorzugsweise 0,02 bis 3 Gew.-% einer Verbindung der Formel I enthalten, ausgezeichnete Hochdruck-Schmiereigenschaften, welche durch stark reduzierte Abnutzungserscheinungen der zu schmierenden Reibpartner deutlich werden. Die in Frage kommenden Schmiermittel sind dem Fachmann geläufig. Beispiele für solche Schmiermittel finden sich z. B. im »Schmiermittel Taschenbuch« (Hüthig Verlag, Heidelberg, 1974).

Die Schmierölformulierung kann zusätzlich noch andere Additive enthalten, die zugegeben werden, um gewisse Grundöleigenschaften zu verbessern, wie Antioxidantien, Metallpassivatoren, Rostinhibitoren, Viskositätsindex-Verbesserer, Stockpunkterniedriger, Dispersants-Detergents und andere Verschleißschutz-Additive.

Beispiele für Antioxidantien sind:

a) Alkylierte und nicht-alkylierte aromatische Amine und Mischungen davon, z. B.: Dioctyldiphenylamin, Mono-tert.-octylphenyl-α- und -β-naphthylamine, Phenothiazin, Dioctylphenothiazin, Phenyl-α-naphthylamin, N,N'-Di-sec.-butyl-p-phenylendiamin.
b) Sterisch gehinderte Phenole, z. B. 2,6-Di-tert.-butyl-p-cresol, 4,4'-Bis-(2,6-diisopropylphenol), 2,4,6-Triisopropylphenol, 2,2'-Thio-bis-(4-methyl-6-tert.-butyl-phenol), 4,4'-Methylen-bis-(2,6-di-tert.-butylphenol).
c) Alkyl-, Aryl- oder Alkaryl-phosphite, z. B.: Trinonylphosphit, Triphenylphosphit, Diphenyldecylphosphit.
d) Ester von Thiodipropionsäure oder Thiodiessigsäure, z. B.: Dilaurylthiodipropionat oder Dioctylthiodiacetat.
e) Salze von Carbamin- und Dithiophosphorsäuren, z. B.: Antimon-diamyldithiocarbamat, Zink-diamyldithiophosphat.
f) Kombinationen von zwei oder mehr Antioxidantien der obigen, z. B.: ein alkyliertes Amin und ein sterisch gehindertes Phenol.

Beispiele für Metallpassivatoren sind:

a) für Kupfer z. B. Benzotriazol, Tetrahydrobenzotriazol, 2-Mercapto-benzothiazol, 2,5-Dimercaptothiadiazol, Salicylidenpropylendiamin, Salze von Salicylaminoguanidin.
b) für Blei z. B. Sebacinsäurederivate, Chinizarin, Propylgallat.
c) Kombination von zwei oder mehr der obigen Additive.

Beispiele für Rostinhibitoren sind:

a) Organische Säuren, ihre Ester, Metallsalze und Anhydride, z. B.: N-Oleoyl-sarcosin, Sorbitan-mono-oleat, Blei-naphthenat, Dodecenyl-bernsteinsäure-anhydrid.
b) Stickstoffhaltige Verbindungen, z. B.:

I. primäre, sekundäre oder tertiäre aliphatische oder cycloaliphatische Amine und Aminsalze von organischen und anorganischen Säuren, z. B. öllösliche Alkylammoniumcarboxylate.
II. Heterocyclische Verbindungen z. B.: substituierte Imidazoline und Oxazoline.

c) Phosphorhaltige Verbindungen, z. B.: Aminsalze von Phosphorsäurepartialestern.
d) Schwefelhaltige Verbindungen, z. B.: Barium-dinonylnaphthalin-sulfonate, Calcium-petroleumsulfonate.
e) Kombinationen von zwei oder mehr der obigen Additive.

Beispiele für Viskositätsindex-Verbesserer sind: Polymethacrylate, Vinylpyrrolidon/Methacrylat-Copolymere, Polybutene, Olefin-Copolymere, Styryl/Acrylat-Copolymere.

Beispiele für Stockpunkterniedriger sind: Polymethacrylate, alkylierte Naphthalinderivate.

Beispiele für Dispersants/Detergents sind: Polybutenylbernsteinsäure-imide, Polybutenylphosphonsäurederivate, überbasische Magnesium-, Calcium- und Bariumsulfonate und -phenolate.

Beispiele für andere Verschleißschutz-Additive sind: Schwefel und/oder Phosphor und/oder Halogen enthaltende Verbindungen, wie geschwefelte vegetabilische Öle, Zinkdialkyldithiophosphate, Tritolyl-phosphat, chlorierte Paraffine, Alkyl- und Aryldisulfide.

Die folgenden Beispiele erläutern die Erfindung.

## Beispiel 1

### Stabilisierung von Acrylnitril-Butadienstyrol (ABS)

0,7 g 3,5-Di-tert.-butyl-4-hydroxybenzyl-mercapto-essigsäure-2-äthyl-n-hexylester werden in 40 ml Hexan gelöst. Die Lösung wird unter kräftigem Rühren zu einer Dispersion von 100 g ABS in 600 g Wasser gegeben. Die Antioxidans-Lösung wird durch das ABS in kurzer Zeit (1 Minute) vollständig absorbiert. Das ABS-Pulver wird abgenutscht und während 40 Stunden bei 40°C im Vakuum getrocknet. Dem trockenen Pulver werden 2% Titandioxid (Pigment), sowie 1% Äthylen-bis-stearinsäure-amid (Gleitmittel) zugegeben. Die Mischung wird anschließend während 4 Minuten auf einem Zweiwalzenstuhl bei 180°C compoundiert. Aus dem Walzfell wird bei 175°C eine Platte von 0,8 mm Dicke gepreßt, aus welcher Prüflinge der Dimension 45 × 17 mm ausgestanzt werden.

Die Platte ohne die erfindungsgemäße Verbindung wird auf dieselbe Weise hergestellt.

Die Prüfung auf Wirksamkeit des zugesetzten Additivs wird durch Hitzealterung in einem Umlaufofen bei 200° C vorgenommen. Als Kriterium dient die Farbentwicklung nach 30 Minuten Prüfdauer. Die Farbintensität wird mit dem »Yellowness Index« nach ASTM D 1925 quantifiziert. Höhere Zahlen bedeuten intensiveren Gelbeindruck. Die Versuche zeigen, daß die Farbentwicklung durch das zugesetzte Additiv wirksam unterdrückt wird.

| | Yellowness Index nach 30 Minuten |
|---|---|
| Ohne Stabilisator | 47 |
| 0,7% 3,5-di-tert.-butyl-4-hydroxibenzyl-mercaptoessigsäure-2-äthyl-n-hexylester | 36 |

## Beispiel 2

### Stabilisierung von Polybutadien-Kautschuk

100 Teile Polybutadien, welches mit 0,36% 2,6-Di-tert.-butyl-p-cresol vorstabilisiert ist, werden zusätzlich mit 0,05, 0,1 und 0,2% 3,5-Di-tert.-butyl-4-hydroxybenzyl-mercapto-essigsäure-2-äthyl-n-hexylester in einem Brabender-Plastographen bei 150° C und 60 U/min während 30 Minuten geknetet.

Als Kriterium für die Wirksamkeit des zugesetzten Additivs dient der nach der Brabender-Verarbeitung auftretende Gelgehalt des Materials. Dieser wird folgendermaßen bestimmt: 1 Gramm Polybutadien wird über Nacht bei Raumtemperatur in 100 ml Toluol gelöst. Die Lösung wird durch Glaswolle filtriert und die filtrierte Lösung zur Trockene eingedampft. Der Gelgehalt ergibt sich aus:

$$\text{Gel} = \frac{E-A}{E} \times 100\,(\%)$$

E = Einwaage (1 Gramm)
A = Gewicht des Eindampfrückstandes

Der Versuch zeigt die wirksame Unterdrückung der Gelentwicklung während der Verarbeitung durch das zugesetzte Additiv.

| | Gelgehalt % |
|---|---|
| Ohne Stabilisator | 50 |
| 3,5-Di-tert.-butyl-4-hydroxibenzyl-mercaptoessigsäure-2-äthyl-n-hexylester | |
| 0,05% | 22 |
| 0,1% | 12 |
| 0,2% | 5 |

## Beispiel 3

### Stabilisierung von Polybutadien-Kautschuk

100 Teile Polybutadien, welches mit 0,36% 2,6-Di-tert.-butyl-p-cresol vorstabilisiert ist, werden zusätzlich mit 0,1; bzw. 0,2% 3,5-Di-tert.-butyl-4-hydroxibenzyl-mercapto-essigsäure-2-äthyl-n-hexylester auf einem Zweiwalzenstuhl bei 50° C während 6 Minuten plastifiziert.

Aus den Walzfellen werden bei 60° C Platten von 10 mm Dicke gepreßt. Die Platte ohne die erfindungsgemäße Verbindung wird auf dieselbe Weise hergestellt.

Die Prüfung auf Wirksamkeit des zugesetzten Additivs wird durch Hitzealterung in einem Umluftofen bei 80° C vorgenommen. Als Kriterium dient der während der Ofenalterung auftretende Gelgehalt. (Bestimmung wie im Beispiel 2). Der Gelgehalt nimmt nach einer Induktionsperiode rasch zu. Als willkürliche Definition der Induktionsperiode dient die Zeit, nach welcher ein Gelgehalt von 5% erreicht wird.

Der Versuch zeigt, daß die Induktionszeiten durch das zugesetzte Additiv wirksam verlängert werden können.

| | Induktionszeit in Wochen bis Gel = 5% erreicht |
|---|---|
| Ohne Addition | 3 |
| 3,5-Di-tert.-butyl-4-hydroxi-benzyl-mercaptoessigsäure-2-äthyl-n-hexylester | |
| 0,1% | 7 |
| 0,2% | 11 |

Beispiel 4

Stabilisierung von Polybutadien-Kautschuk

Die Arbeitsweise des Beispiels 3 wird wiederholt mit dem Unterschied, daß ein Material verwendet wird, das von der Fabrikation her keinen Stabilisator enthält.
Als Induktionsperiode wird die Zeit definiert, nach welcher ein Gelgehalt von 1% erreicht wird.

| | Induktionszeit in Tagen bis Gel = 1% erreicht |
|---|---|
| Ohne Stabilisator | 10 |
| 3,5-Di-tert.-butyl-4-hydroxi-benzyl-mercaptoessigsäure-2-äthyl-n-hexylester | |
| 0,2% | 48 |

Beispiel 5

Stabilisierung von Polybutadien-Kautschuk

Die Prüflinge des Beispiels 4 werden zudem durch Eintauchen in Siliconöl bei 160°C während 20 Minuten gealtert. Als Kriterium dient der Gelgehalt am Ende der Alterung.
Die Versuche zeigen die wirksame Unterdrückung der Gelentwicklung während der Alterung durch das zugesetzte Additiv.

| | Gelgehalt, % |
|---|---|
| Ohne Additiv | 88 |
| 3,5-Di-tert.-butyl-4-hydroxi-benzyl-mercaptoessigsäure-2-äthyl-n-hexyl-ester 0,2% | 6 |

Beispiele 6 bis 13

Substrat: vorstabilisiertes BR Solprene 250® (Phillips Petroleum). Einarbeitung und Probenherstellung: Die Additive werden bei 50° C auf einem Mischwalzwerk eingearbeitet. Dann werden bei 80° C in einer hydraulischen Heizpresse 2- und 10-mm-Platten gepreßt.

Alterungen:

a) Ofenalterung bei 80° C mit 10-mm-Platten in einem HORO-Umluftofen; periodische Gelbestimmung bei Raumtemperatur in Toluol.
b) Alterung in einem Brabender-Plasticorder bei 160° C, 60 rpm für 30 Minuten. Bestimmung der Induktionszeit ($T_{ind}$) in Minuten (Zeit bis zum Anstieg des Drehmomentes um 100 Meterpond über das Minimum); Gelbestimmung in Toluol nach Alterung; Farbmessung von 2-mm-Proben des gealterten Materials.
c) Alterung von 2-mm-Proben bei 160° C für 30 Minuten in Siliconöl; Bestimmung des Gelgehaltes bei Raumtemperatur in Toluol.
d) Bestimmung der Verfärbung durch Messung vom Yellownessindex nach ASTM D 1925-70 während 10 Wochen Lagerung von 2-mm-Proben A) am diffusen Tageslicht, B) im Dunkeln und C) bei 80° C im Umluftofen.

Ergebnisse der Alterungsversuche

| Beispiel Nr. | Stabilisator | Konz. % | Ofen 80°C Wochen bis Gel >5% | Brabender 160°C 60 rpm, 30 Min. $T_{ind}$ (Min.) | Gel % | YI | Gel in % nach Siliconöl-alterung bei 160°C, 30′ | max. Yellownessindex bei 10 Wochen Lagerung Tages-licht | Dunkel-heit | Ofen 80°C |
|---|---|---|---|---|---|---|---|---|---|---|
| 6 | 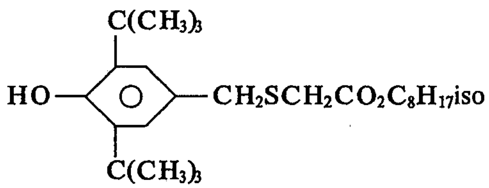 | 0,1 | 8 | 11,3 | 43 | 31 | 4,8 | 9 | 9 | 110 |
| | | 0,25 | 13 | 14,8 | 29 | 25 | 4,4 | 9 | 8 | 34 |
| | | 0,5 | 16 | 17,5 | 19 | 16 | 2,8 | 9 | 9 | 17 |
| 7 | 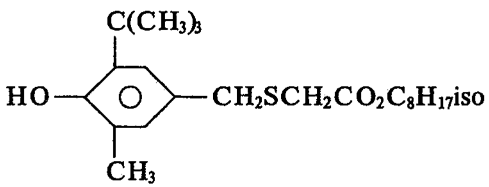 | 0,1 | 8 | 9,3 | 50 | 28 | 4,9 | 10 | 10 | 98 |
| | | 0,25 | 10 | 8,5 | 43 | 34 | 4,3 | 11 | 9 | 36 |
| | | 0,5 | 12 | 8,0 | 47 | 32 | 2,2 | 9 | 10 | 27 |
| 8 | 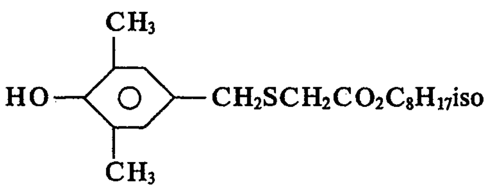 | 0,1 | 7 | 9,3 | 51 | 33 | 7,4 | 10 | 9 | 108 |
| | | 0,25 | 7 | 6,5 | 48 | 38 | 5,1 | 10 | 9 | 35 |
| | | 0,5 | 7 | 5,8 | 51 | 38 | 3,4 | 9 | 9 | 27 |
| 9 | 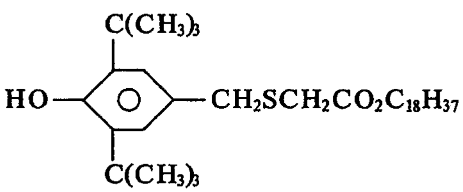 | 0,1 | 8 | 10,8 | 47 | 29 | 9,4 | 10 | 11 | 128 |
| | | 0,25 | 12 | 12,5 | 37 | 27 | 5,6 | 11 | 10 | 83 |
| | | 0,5 | 16 | 14,0 | 21 | 25 | 3,1 | 10 | 8 | 20 |

Fortsetzung

| Beispiel Nr. | Stabilisator | Konz. % | Ofen 80°C Wochen bis Gel >5% | Brabender 160°C 60 rpm, 30 Min. $T_{ind}$ (Min.) | Gel % | YI | Gel in % nach Siliconölalterung bei 160°C, 30′ | max. Yellownessindex bei 10 Wochen Lagerung Tageslicht | Dunkelheit | Ofen 80°C |
|---|---|---|---|---|---|---|---|---|---|---|
| 10 | 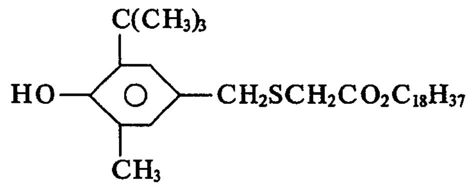 | 0,1 | 8 | 8,3 | 51 | 30 | 11,4 | 8 | 11 | 101 |
| | | 0,25 | 9 | 9,5 | 47 | 32 | 4,3 | 9 | 11 | 75 |
| | | 0,5 | 8 | 9,0 | 45 | 31 | 1,2 | 11 | 10 | 34 |
| 11 | 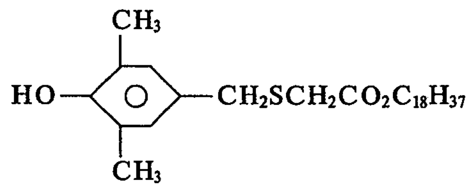 | 0,1 | 6 | 7,8 | 55 | 37 | 11,5 | 13 | 15 | 100 |
| | | 0,25 | 6 | 6,8 | 59 | 37 | 9,2 | 13 | 14 | 76 |
| | | 0,5 | 7 | 7,3 | 56 | 37 | 9,6 | 14 | 14 | 33 |
| 12 (zum Vergleich) | 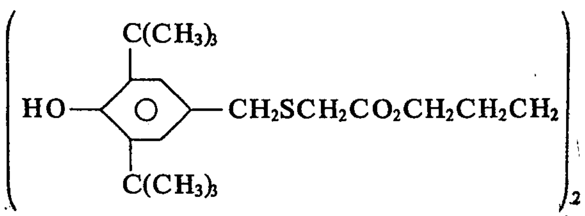 | 0,1 | 6 | 11,5 | 53 | 29 | 2,0 | 11 | 9 | 115 |
| | | 0,25 | 8 | 11,5 | 52 | 32 | 1,2 | 12 | 10 | 59 |
| | | 0,5 | 12 | 11,0 | 52 | 35 | 0,7 | 12 | 9 | 25 |
| 13 (zum Vergleich) | 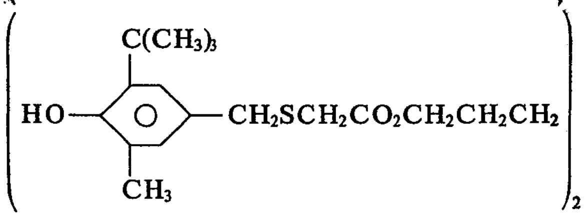 | 0,1 | 8 | 9,5 | 47 | 30 | 2,2 | 10 | 10 | 96 |
| | | 0,25 | 9 | 9,5 | 51 | 32 | 0,5 | 10 | 11 | 30 |
| | | 0,5 | 10 | 9,5 | 50 | 31 | 0,2 | 12 | 12 | 23 |
| Ohne (Kontrolle) | | – | 6 | 8,8 | 58 | 33 | 12,3 | 10 | 8 | 102 |

Beispiel 14

Wirkung von 3,5-Di-tert.-butyl-4-hydroxy-benzyl-mercaptoessigsäure-2-äthyl-n-hexylester in vorstabilisiertem Polyisopren.

Substrat:
Cariflex IR 305® (enthält ca. 0,13% BHT) (Shell)

Einarbeitung:
Walzwerk bei 60° C

Probenherstellung:
Platten von 10 mm Dicke bei 90° C, 15 Minuten gepreßt

Alterung:
Umluftofen bei 70° C

Alterungskriterium:
Veränderung der Mooneyviskosität (Abfall).

| | Mooneyviskosität ML 1′ + 4′ (100° C) nach Tagen 0 | 7 | 14 | 21 | 28 | 35 |
|---|---|---|---|---|---|---|
| Ohne Stabilisator | 69 | 49 | 36 | 25 | | |
| 0,2% Stabilisator | 69 | 60 | 50 | 50 | 41 | 39 |

Beispiel 15

Wirkung von 3,5-Di-tert.-butyl-4-hydroxybenzyl-mercaptoessigsäure-2-äthyl-n-hexylester in unstabilisiertem, emulsionspolymerisiertem SBR.

Substrat:
emulsionspolymerisierter SBR-Latex Polysar® (Shell)

Einarbeitung:
Lösen der Stabilisierung in wenig Aceton und vorsichtiges Einrühren in den Latex.

Koagulation:
Eintropfen vom Latex in 0,35%ige wäßrige $CaCl_2$-Lösung unter starkem Rühren bei 90° C; 2mal mit dest. Wasser waschen; im Vakuum bei 40° C trocknen.

Probenherstellung:
Platten von 10 mm Dicke bei 80° C, 15 Minuten gepreßt.

Alterung:
Ofen bei 80° C

Alterungskriterium:
Anstieg der Mooneyviskosität.

| | Mooneyviskosität ML 1′ + 4′ (100° C) nach Wochen | | | | | | |
|---|---|---|---|---|---|---|---|
| | 0 | 1 | 2 | 3 | 4 | 5 | 6 |
| Ohne Stabilisator | 55 | 60 | 68 | 94 | | | |
| 0,3% des oben angegebenen Stabilisators | 54 | 53 | 52 | | 55 | 55 | 74 |
| 0,3% Wingstay L® 1) | 54 | 55 | 61 | 72 | 85 | | |
| 1,25% Wingstay S® 2) | 56 | 80 | 92 | 107 | | | |
| 0,75% Naugawhite liq® 3) | 56 | 53 | 58 | 65 | 78 | 85 | |

1) Stabilisator auf der Basis gehinderte Phenole der Firma Goodyear.
2) Phenolstyrol-Stabilisator der Firma Goodyear.
3) Alkylbisphenol im Trägermaterial der Firma Naugatuck.

Beispiel 16

Wirkung von 3,5-Di-tert.-butyl-4-hydroxybenzyl-mercaptoessigsäure-2-äthyl-n-hexylester in einem SBS Schuhsohlencompound.

Rezeptur:

| | |
|---|---|
| SBS Europrene T 171® (100 Teile SBS + 50 Teile Öl) der Firma Anic: | 150 Gewichtsteile |
| Kristallpolystyrol N 168® (BASF) | 30 Gewichtsteile |
| Oel Talpa 945® (Shell) | 20 Gewichtsteile |
| Ultrasil VN 3® (Bayer) | 10 Gewichtsteile |

Herstellung der Mischung und Einarbeitung der Stabilisatoren:
auf der Walze bei 160° C.

Probenherstellung:
Pressen von 0,5-mm-Platten bei 130° C, 10 Minuten; Ausstanzen von Schulterstäben ISO S 2.

Alterung:
Zellenofen bei 70° C.

Prüfung:
Bestimmung von Bruchfestigkeit (kp/cm²) und Dehnung (%) durch Zugprüfversuch

| Stabilisator | Bruchfestigkeit in kp/cm² nach Wochen | | | | | Dehnung in % nach Wochen | | | | |
|---|---|---|---|---|---|---|---|---|---|---|
| | 0 | 4 | 6 | 10 | 14 | 0 | 4 | 6 | 10 | 14 |
| ohne | 101 | 30 | | | | 640 | 300 | | | |
| 0,2% des obigen Stabilisators | 110 | 101 | 99 | 96 | 83 | 650 | 600 | 585 | 570 | 550 |
| 0,2% 3,4-Di-tert.-butyl-4-hydroxy-benzylmercaptoessigsäure-methylester (zum Vergleich) | | | | | | | | | | |
| 0,4% Topanol OC® | 104 | 100 | 99 | 88 | 80 | 620 | 600 | 590 | 530 | 520 |
| 0,8% Trinonylphenylphosphit | 95 | 78 | 64 | 40 | | 620 | 252 | 465 | 330 | |

In Beispiel 17 wird die Herstellung einer erfindungsgemäß verwendeten Verbindung der Formel I näher erläutert.

Beispiel 17

3,5-Di-tert.-butyl-4-hydroxy-benzyl-mercaptoessigsäure-2-äthyl-n-hexyl-ester

26,3 g 2,6-Di-tert.-butylbenzyl-dimethylamin, 20,4 g Thioglykoläthyl-n-hexylester und 50 mg Dimethylformamid werden unter Stickstoff und Rühren 2 Stunden bei 120° C erhitzt. Es entweicht Dimethylamin. Im Dünnschichtchromatogramm (D. C.) sind nunmehr praktisch keine Edukte mehr nachweisbar. Das Lösungsmittel wird im Vakuum entfernt und der viskose Rückstand in 100 ml Ligroin gelöst. Nach der Wäsche mit Wasser wird die getrocknete Ligroinlösung durch Behandeln mit 1 g Kieselgur »Hyflow Supercel« (Jons Manville Sales Corp.) entfärbt, klar filtriert und das Lösungsmittel i. V. entfernt.

Man erhält 38,5 g des Produktes dieses Beispiels in Form eines fast farblosen viskosen Öls, welches bei längerem Stehen bei ca. 20° C kristallin erstarrt und welches durch Elementar-Analyse und D. C. definiert ist.

Ergebnis der Elementar-Analyse: $C_{25}H_{42}O_3S$ (422,67)

| | | |
|---|---|---|
| C | ber. 71,4; | gef. 71,00 |
| H | ber. 10,02; | gef. 9,90 |
| S | ber. 7,59; | gef. 7,70 |

Beispiel 18

Es wird der Öl-Oxidationstest, Standard Version nach ASTM D 2272 (Rotary Bomb Oxidation Test), in folgender Weise durchgeführt. Eine Ölprobe von 50 ml Mineralöl Marke Vitrea 100® der Firma SHELL wird unter Zusatz von 0,25 g des Stabilisators gemäß Beispiel 17 in einem Glasgefäß zusammen mit 5 ml dest. Wasser und einer blankpolierten, mit Petroläther gewaschenen katalytisch wirkenden Cu-Spirale in einer Sauerstoff-Atmosphäre oxydiert.

Das Glasgefäß befindet sich in einer Bombe aus rostfreiem Stahl mit Manometer. Die Bombe dreht sich axial mit 100 U/Min. in einem Winkel von 30° zur Horizontalen in einem Ölbad bei 150°C. Der Sauerstoffdruck beträgt anfangs, vor dem Aufheizen, ca. 6 bar, steigt bei 150° C auf knapp 14 bar und bleibt bis zur einsetzenden Oxidation konstant. Die Prüfung ist beendet bei einem Druckabfall um 1,7 bar. Aufgezeichnet wird die Zeit in Minuten.

Ergebnis:

| Stabilisator | Minuten bis Druckabfall um 1,7 bar |
|---|---|
| ohne | 16 |
| gemäß Beispiel 17 | 101 |

Beispiel 19

Es wird der Öl-Oxidationstest in modifizierter Version nach IP 280, »CIGRE« durchgeführt.

Bedingungen:
4 Stunden Sauerstoff einleiten bei 150° C (4 Liter $O_2$/h).

Bestimmung der Säurezahl nach Testende; Tabellenwert:
mg KOH-Verbrauch pro g Testöl.

Stabilisatorkonzentration:
0,5 Gew.-%

Testöl:
Mineralöl der Marke »Vitrea 100« der Firma SHELL.

Ergebnis:

| Stabilisator | mg KOH/g |
|---|---|
| ohne | 3,6 |
| gemäß Beispiel 17 | 0,59 |

**Patentansprüche**

1. Organische Elastomere sowie mineralische und synthetische Schmieröle, enthaltend als Stabilisator einen Phenol-mercaptocarbonsäureester der Formel I

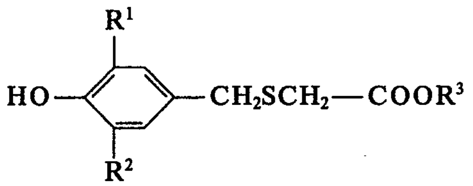

(I)

worin $R^1$ tert.-Butyl, $R^2$ Methyl oder tert.-Butyl und $R^3$ geradkettiges oder verzweigtes, ununterbrochenes oder durch —O— oder —S— unterbrochenes $C_4$—$C_{18}$-Alkyl, $C_5$—$C_6$-Cycloalkyl oder Benzyl bedeuten.

2. Organische Elastomere gemäß Anspruch 1, dadurch gekennzeichnet, daß sie als Stabilisator einen Phenol-mercaptocarbonsäureester der Formel II

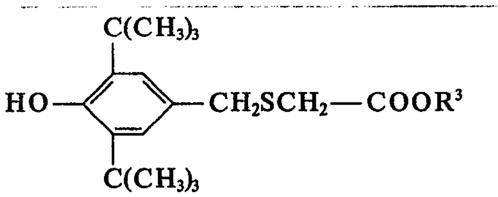

(II)

worin $R^3$ $C_5$—$C_{12}$-Alkyl ist, enthalten.

3. Organische Elastomere gemäß Anspruch 1, dadurch gekennzeichnet, daß sie als Stabilisator eine Verbindung der Formel III

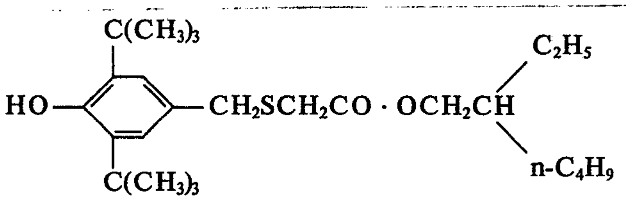

(III)

enthalten.

4. Mineralische und synthetische Schmieröle gemäß Anspuch 1, dadurch gekennzeichnet, daß sie als Stabilisator einen Phenol-mercaptocarbonsäureester der Formel II

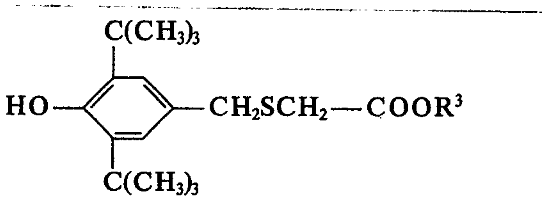

(II)

worin $R^3$ $C_5$—$C_{12}$-Alkyl bedeutet, enthalten.

5. Mineralische und synthetische Schmieröle gemäß Anspruch 1, dadurch gekennzeichnet, daß sie als Stabilisator eine Verbindung der Formel III

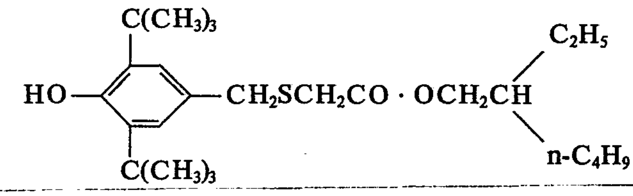

(III)

enthalten.

**Claims**

1. Organic elastomers and mineral and synthetic lubricating oils containing as stabiliser a phenolmercaptocarboxylic acid ester of the formula I

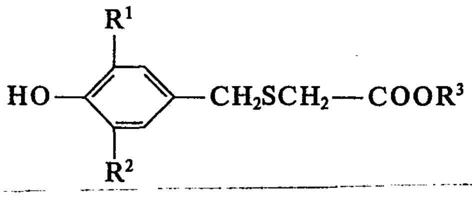

(I)

wherein $R^1$ is tert-butyl, $R^2$ is methyl or tert-butyl and $R^3$ is straight chain or branched $C_4-C_{18}$alkyl which may be interrupted by —O— or —S—, or it is $C_5-C_6$cycloalkyl or benzyl.

2. Organic elastomers according to claim 1, wherein the stabiliser is a phenolmercaptocarboxylic acid ester of the formula II

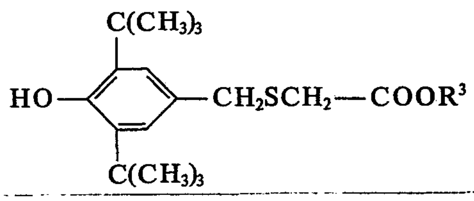

(II)

wherein $R^3$ is $C_5-C_{12}$alkyl.

3. Organic elastomers according to claim 1, wherein the stabiliser is a compound of the formula III

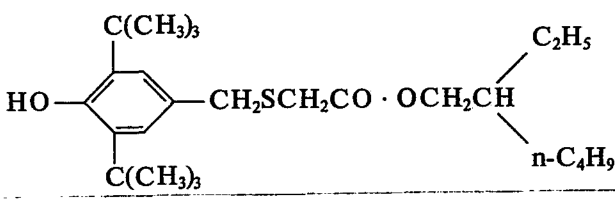

(III)

Mineral and synthetic lubricating oils according to claim 1, wherein the stabiliser is a phenolmercaptocarboxylic acid ester of the formula II

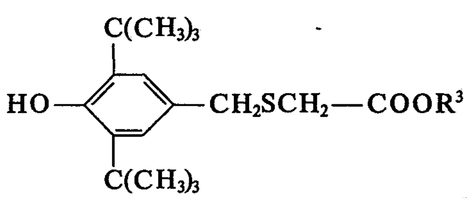

(II)

wherein $R^3$ is $C_5-C_{12}$alkyl.

5. Mineral and synthetic lubricating oils according to claim 1, wherein the stabiliser is a compound of the formula III

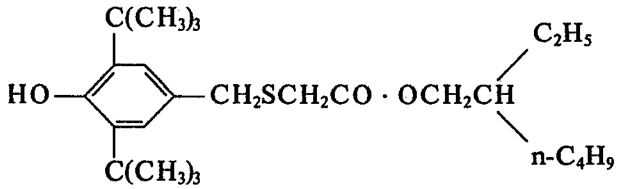

(III)

**Revendications**

1. Elastomères organiques et huiles lubrifiantes minérales ou synthétiques qui contiennent, comme stabilisant, un ester d'acide phénol-mercaptocarboxylique répondant à la formule I:

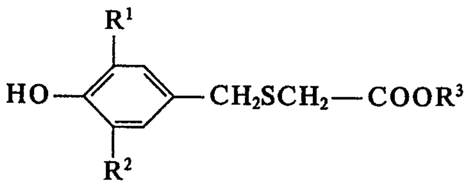

(I)

dans laquelle $R^1$ représente un radical tert-butyle, $R^2$ un radical méthyle ou tert-butyle et $R^3$ un radical alkyle en $C_4-C_{18}$ linéaire ou ramifié, non interrompu ou interrompu par —O— ou —S—, un radical cycloalkyle en $C_5$ ou $C_6$ ou un radical benzyle.

2. Elastomères organiques selon la revendication 1, caractérisés en ce qu'il contiennent, comme stabilisant, un ester d'acide phénol-mercaptocarboxylique répondant à la formule II:

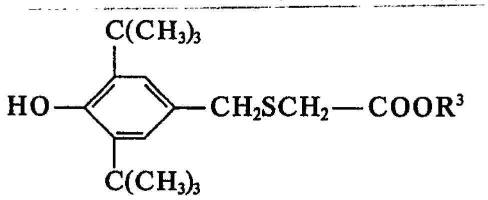

(II)

dans laquelle $R^3$ représente un radical alkyle contenant de 5 à 12 atomes de carbone.

3. Elastomères organiques selon la revendication 1, caractérisés en ce qu'ils contiennent, comme stabilisant, un composé de formule III:

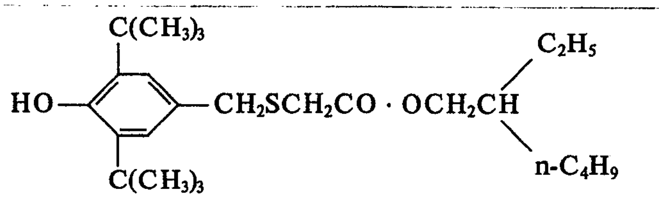

(III)

4. Huiles lubrifiantes minérales ou synthétiques selon la revendication 1, caractérisées en ce qu'elles contiennent, comme stabilisant, un ester d'acide phénol-mercaptocarboxylique qui répond à la formule II.

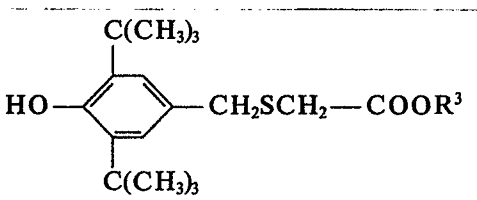

(II)

dans laquelle $R^3$ représente un radical alkyle contenant de 5 à 12 atomes de carbone.

5. Huiles lubrifiantes minérales ou synthétiques selon la revendication 1, qui contiennent, comme stabilisant, un composés de formule III:

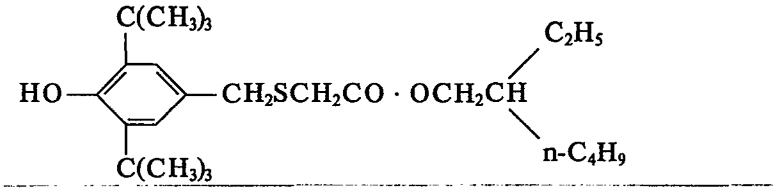

(III)